# EUROPEAN PATENT SPECIFICATION

(11) **EP 2 992 864 B1**
(45) Date of publication and mention of the grant of the patent: **21.04.2021**
(21) Application number: 14183772.4
(22) Date of filing: 05.09.2014
(51) Int. Cl.: A61F 13/475, A61F 13/494, A61F 13/537

(54) **Absorbent structure with edge barrier**
Absorbierende Struktur mit Kantenbarriere
Structure absorbante avec barrière de bord

(43) Date of publication of application: 09.03.2016
(73) Proprietor: Drylock Technologies N.V., 9240 Zele (BE)
(72) Inventor: Van Ingelgem, Werner, 9240 Zele (BE)
(74) Representative: D'Halleweyn, Nele Veerle Trees Gertrudis

(56) References cited:
- EP-A1- 2 740 449
- WO-A1-93/06804
- US-A1- 2006 020 250

## Description

### TECHNICAL FIELD

The invention pertains to the technical field of absorbent articles, such as diapers, baby pants, adult incontinent garments, feminine hygiene garments and the like, and to absorbent structures for use in such absorbent articles.

### BACKGROUND

Absorbent articles such as diapers, baby pants, adult incontinent garments and the like, typically comprise an absorbent structure, positioned in between a topsheet and a backsheet, which comprises an absorbent material that is able to absorb the liquid bodily excretions of the user of the absorbent article. The absorbent material of such absorbent structure is typically an absorbent particulate polymer material which is usually dispersed in a matrix of cellulose fibers or fluff pulp in order to prevent the particulate material from aggregating, as well as to prevent gel blocking. Gel blocking can occur when the absorbent particulate polymer material absorbs liquid, as they tend to typically swell and form a gel structure. This gel structure often blocks the further transfer of liquid into the remaining absorbent structure. As a result, the liquid may be unable to reach the remaining absorbent particulate polymer material and the efficiency of the absorbent article decreases significantly.

Recent years, there has been a trend to produce absorbent articles of which the absorbent structure contains little to no cellulose fibers or fluff pulp, as the latter tend to be quite bulky, thus rendering generally more thick absorbent structures which reduces the overall wearing comfort of the user of the absorbent article. On the other hand, when using absorbent structures with little to no cellulose fibers or fluff pulp, problems such as increased fluid leakage are often encountered and it has also been shown to be difficult to fully, instantly and timely utilize the entire absorption capacity of the absorbent structure, often leading to dysfunctional products and/or bad rewet values.

Furthermore, an additional problem which is being encountered with such structure is related to the migration, loss and leakage of the absorbent particulate polymer material from the absorbent structure during dry and/or wet state, which leads to irritation, skin problems and overall discomfort for the user. Furthermore, it can also lead to dysfunctional products due to lowered uptake capacity, gel blocking, enhanced rewet values and the creation of ruptures or pinholes through the liquid pervious topsheet and/or liquid impervious backsheet of such absorbent articles during manufacturing, high pressured packaging, transportation and storing, but also regular and prolonged usage by the user and/or caregiver.

EP 2 740 449 A1 discloses an absorbent article for personal hygiene such as a diaper or training pant having a front edge and a back edge. The article has a length L as measured along the longitudinal axis from its front edge to its back edge, and a crotch point (C) defined as the point placed at a distance of two fifth of L from the front edge of the article on the longitudinal axis. The article comprises a liquid permeable topsheet, a liquid impermeable backsheet, a pair of barrier leg cuffs extending at least partially between the front edge and the back edge of the diaper on opposite sides of the longitudinal axis and present at least at the longitudinal position of the crotch point, each barrier leg cuff being delimited by a proximal edge joined directly or indirectly to the topsheet and/or the backsheet and a free terminal edge, and an absorbent core comprising a core wrap enclosing an absorbent material, wherein the absorbent material comprises at least 80% of superabsorbent polymers by weight of the absorbent material, wherein the absorbent core comprises at least one channel at least partially oriented in the longitudinal direction of the article. There remains a need in the art for improved absorbent articles comprising an absorbent structure that comprises little to no fluff pulp or cellulose fibers that do not display the above problems and of which the absorbent article capacity, fit and comfort can fully be utilized.

The present invention aims to resolve at least some of the problems mentioned above.

The invention thereto aims to provide an absorbent article of which the absorbent structure is provided with an edge barrier which can reduce at least some of the above problems and can more optimally utilize the absorption, acquisition, distribution and retention capacity of the absorbent structure.

### SUMMARY OF THE INVENTION

The present invention provides an absorbent article comprising an essentially fluffless absorbent structure with a substantially liquid impermeable and/or substantially absorbent particulate polymer material impermeable edge barrier as described in claim 1.

Such edge barrier can prevent leakage of fluids and/or loss of absorbent particulate polymer material along at least one of the edges of the absorbent structure and is capable of blocking, transporting, redirecting and/or redistributing the bodily fluids within the absorbent structure so as to more optimally utilize the functional and structural capacity of the absorbent structure. In an embodiment according to the present invention, the absorbent structure also comprises a substantially liquid impermeable and/or substantially absorbent particulate polymer material impermeable wicking layer which faces the backsheet of the absorbent article and extends along at least a part of the length and width of the absorbent structure, which further contributes to the prevention of dysfunctional absorbent articles and to the transporting, redirecting and/or distributing of liquids within the absorbent structure.

In the preferred embodiment according to the present invention, the absorbent structure comprises an absorbent core, which absorbent core comprises at least one essentially fluffless fibrous substrate layer in which absorbent particulate polymer material is dispersed and/or embedded. The substrate layer hereby aids in immobilizing the absorbent particulate polymer material and preferably also helps in distributing liquid across the absorbent structure, further contributing to an optimal use of the functional capacities thereof.

In a second aspect, the present invention provides an absorbent structure as is described in claim 17, and an absorbent structure for use in an absorbent article according to claim 1.

### DESCRIPTION OF FIGURES

**Figure 1** is a top plan view of a diaper as a preferred embodiment of an absorbent article according to the present invention.
**Figure 2** shows a schematic side view of the absorbent article of Figure 1 cut along line A-A, hereby showing the components of an absorbent structure according to a preferred embodiment of the current invention.
**Figure** 3 provides cross-sectional schematic illustrations of absorbent structures according to various embodiments of the current invention.
**Figure 4** provides a top view schematic illustration of different configurations of absorbent structures according to various embodiments of the current invention.
**Figure 5A** displays a schematic top plan view of an absorbent structure according to another preferred embodiment according to the present invention and **Figure 5B** shows a schematic side view of the absorbent structure of Figure 4B showing its various components.

### DETAILED DESCRIPTION OF THE INVENTION

The present invention concerns an absorbent article which comprises an essentially fluffless absorbent structure that is provided with a substantially liquid impermeable and/or substantially absorbent particulate polymer material impermeable edge barrier, thereby reducing the above functional and structural problems associated with the absorbent structure and allowing the absorption capacity of the absorbent structure to be more optimally used.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "an edge barrier" refers to one or more than one edge barrier.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to absorb and/or retain bodily exudates.
"Absorbent component" as used herein refers to a structural constituent of an absorbent structure, e.g., a piece of an absorbent core, such as one of multiple pieces in a multi-piece absorbent core.
"Absorbent element" as used herein refers to a part of a functional constituent of an absorbent structure, e.g., a liquid acquisition layer, a liquid distribution layer, or a liquid storage layer formed of a material or materials having particular liquid handling characteristics suitable for the specific function.
"Absorbent insert" as used herein refers to a device adapted for insertion into an absorbent article and to serve as an absorbent structure when so inserted.
"Absorbent layer" as used herein refers to a term referring to a discrete, identifiable sheet-like or web-like element of an absorbent structure which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g bond area's) or unintentional (e.g. manufacturing artefacts).
"Absorbent particulate polymer material" as used herein refers to an absorbent polymer material which is in particulate form such as powders, granules, flakes and the like so as to be flowable in the dry state.
"Absorption" as used herein refers to the process by which a liquid is taken up within a material.
"Acquisition layer", "acquisition region", "acquisition surface" or "acquisition material" and the like as used herein refer to a layer having a faster liquid uptake capability.
"Absorbency" is the ability of a material to take up fluids by various means including capillary, osmotic, solvent, chemical or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Airlaying" as used herein refers to forming a web by dispersing fibres or particles in an air stream and condensing them from the air stream onto a moving screen by means of a pressure or vacuum; a web of fibres produced by airlaying is herein referred to an "airlaid"; an airlaid web bonded by one or more techniques to provide fabric integrity is herein referred to an "airlaid nonwoven".
"Apparent density", "density" as used herein refers to the basis weight of the sample divided by the calliper with appropriate unit conversions incorporated therein. Apparent density used herein has the unit g/cm³.
"Attach", "attached" and "attachment" as used herein are synonymous with their counterparts of the terms "fasten", "affix", "secure", "glue", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter, g/m² or gsm.
"Bodily exudates", "body exudates", "bodily fluids", "body fluids", "bodily discharges", "body discharges", "liquids" and the like as used herein are used interchangeably and refer to, but are not limited to urine, blood, vaginal discharges, breast milk, sweats and faecal matter.
"Binder", "adhesive", "glue", "resins", "plastics" and the like as used herein are used interchangeably and refer to substances, generally in a solid form (e.g. powder, film, fibre) or as a foam, or in a liquid form (e.g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibres" as used herein refers to naturally occurring fibres based on cellulose, such as, for example cotton, linen, etc; wood pulp fibres are one example of cellulose fibres; man-made fibres derived from cellulose, such as regenerated cellulose (rayon), or partially or fully acetylated cellulose derivatives (e.g. cellulose acetate or triacetate) are also considered as cellulose fibres.
"Cluster" or the like as used herein refers to an agglomeration of particles and/or fibres.
"Chemically stiffened fibres", chemically modified fibres", "chemically cross-linked fibres", "curly fibres" and the like as used herein are used interchangeably and refer to any fibres which have been stiffened by chemical means to increase stiffness of the fibres under both dry and aqueous conditions, for example by way of addition of chemical stiffening agents (e.g. by coating, impregnating, etc), altering the chemical structure of the fibres themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garments, baby diapers and pants and adult incontinence garments.
"Crotch region" of an absorbent article as used herein refers to about 50% of the absorbent article's total length (i.e., in the y-dimension), where the crotch point is located in the longitudinal centre of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them insoluble.
"Diaper", "conventional diaper", "diaper-like", "diaper-like garment" and the like as used herein are used interchangeably and refer to disposable absorbent articles, which typically include a front waist portion and a back waist portion which may be releasably connected about the hips of the wearer during use by conventional fasteners such as adhesive tape fasteners or hook and loop type fasteners. In use, the article is positioned between the legs of the wearer and the fasteners are releasably attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Distribution layer", "distribution region", "distribution surface" or "distribution material" and the like as used herein are used interchangeably and refer to a layer having a larger capacity in wicking, dispersing and distributing liquids.
"Drylaying" as used herein refers to a process for making a nonwoven web from dry fibre; these terms apply to the formation of carded webs, as well as to the air laying formation of random webs; a web of fibres produced by drylaying is herein referred to as a "drylaid"; a drylaid web bonded by one or more techniques to provide fabric integrity is herein referred to a "drylaid nonwoven".
"Dry strength" as used herein refers to the strength of an adhesive joint determined in dry state conditions, immediately after drying under specified conditions or after a period of conditioning in the standard laboratory atmosphere.
"Fabric" as used herein refers to a sheet structure made from fibres, filaments and/or yarns.
"Feminine hygiene garments" as used herein refer to absorbent hygiene articles intended to be worn by woman, for absorbing and containing body exudates.
"Fibre" as used herein refers to the basic threadlike structure from which nonwovens, yarns and textiles are made. It differs from a particle by having a length at least 4 times its width; "Natural fibres" are either of animal (wool, silk), vegetable (cotton, flax, jute) or mineral (asbestos) origin, while "Man-made fibres" may be either polymers synthesised from chemical compounds (polyester, polypropylene, nylon, acrylic etc.) or modified natural polymers (rayon, acetate) or mineral (glass). "Fibre" and "filament" are used interchangeably.
"Fluff pulp" as used herein refers to wood pulp specially prepared to be drylaid.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the backsheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"Highloft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilisation layer" as used herein refers to a layer able to be applied to the absorbent polymer material or absorbent polymer material area with the intent to bond and/or immobilize absorbent material and/or absorbent layer.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibres with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and faces the wearer facing layer; conversely, the term "lower" refers to the layer of the absorbent article which is nearest to and faces the garment facing layer. "Layer" is three dimensional structure with a x dimension width, y dimension length, and z-dimensions thickness or calliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the flow of a liquid from one absorbent element or component to another absorbent element or component by channel flow action.
"Mechanical bonding" as used herein refers to a method of bonding fibres by entangling them. This can be achieved by needling, stitching with fibres or by the use of high-pressure air or water jets and the like.
"Nonwoven" as used herein refers to manufactured sheet, web or batt of directionally or randomly orientated fibres, bonded by friction, and/or cohesion and/or adhesion, excluding paper and products which are woven, knitted, tufted, stitch-bonded incorporating binding yarns or filaments, or felted by wet-milling, whether or not additionally needled. The fibres may be of natural or man-made origin and may be staple or continuous filaments or be formed in situ. Commercially available fibres have diameters ranging from less than about 0.001 mm to more than about 0.2 mm and they come in several different forms: short fibres (known as staple, or chopped), continuous single fibres (filaments or monofilaments), untwisted bundles of continuous filaments (tow), and twisted bundles of continuous filaments (yarn). Nonwoven fabrics can be formed by many processes such as melt blowing, spun bonding, solvent spinning, electrospinning, and carding. The basis weight of nonwoven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Resin" as used herein refers to a solid or semisolid polymeric material.
"Substantially cellulose free" as used herein refers to an absorbent article, structure or core, that contains less than 20% by weight cellulosic fibres, less than 10% cellulosic fibres, less than 5% cellulosic fibres, no cellulosic fibres, or no more than an immaterial amount of cellulosic fibres which do not materially affect the thinness, flexibility or absorbency thereof.
"Thermobonding" as used herein refers to a method of bonding fibres by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localised heat through vibration thereby causing thermoplastic fibres to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibres produced by weaving is herein referred to as a "Woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such web materials may be cellulosic fibre materials, tissues, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, nonwoven/film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of a joint determined immediately after removal from a liquid in which it has been immersed under specified conditions of time, temperature and pressure. The term is commonly used in the art to designate strength after immersion in water.
"Wetlaying" as used herein refers to the forming a web from an aqueous dispersion of fibres by applying modified paper making techniques; a web of fibres produced by wetlaying is herein referred to as a "wetlaid".
"Wood pulp" as used herein refers to cellulosic fibres used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

The recitation of numerical ranges by endpoints includes all numbers and fractions subsumed within that range, as well as the recited endpoints.

In the preferred embodiment the current invention provides an absorbent article comprising a liquid pervious topsheet, a liquid impervious backsheet, and an essentially fluffless absorbent structure positioned in between said backsheet and said topsheet. The absorbent structure comprises a length along an x-axis, a width along a y-axis and a thickness along a z-axis, and further comprises two laterally opposed side edges extending substantially along the x-axis and a front edge and a back edge which extend substantially along the y-axis.

With the term "essentially fluffless absorbent structure" as used herein, it is referred to that the absorbent structure contains less than 20% by weight cellulosic fibres or fluff pulp, less than 10% cellulosic fibres or fluff pulp, less than 5% cellulosic fibres or fluff pulp, no cellulosic fibres or fluff pulp, or no more than an immaterial amount of cellulosic fibres or fluff pulp which do not materially affect the thinness, flexibility or absorbency thereof.

In particular, the absorbent structure of the absorbent article comprises an edge barrier, which edge barrier is substantially liquid impermeable and/or substantially absorbent particulate polymer material impermeable to prevent sideways leakage of fluid and/or absorbent particulate polymer material from the absorbent structure.

"Substantially liquid impermeable" as used herein refers to an edge barrier (or wicking layer) that comprises a material that will substantially prevent the passage of liquids through it. It should be noted that the material can be breathable so as to permit the passage of gasses or vapors, but not of liquids, or the material can be configured so as to prevent the passage of liquids as well as gasses or vapors. The degree of impermeability of a material is typically measured by the hydrostatic head. The term "hydrostatic head" as used herein refers to a way of measuring how waterproof a piece of fabric or material is. The person measuring the impermeability will hereby typically take a clear tube and clamp the material to be measured over the bottom end and will then fill the tube slowly with water and watch to see how high (measured in mm) the column of water can get before the material lets water drip through. In a preferred embodiment, the edge barrier comprises a hydrostatic head of at least 100 mm, more preferably a hydrostatic head of at least 200 mm, most preferably a hydrostatic head of at least 300 mm.

"Substantially absorbent particulate polymer material impermeable" as used herein refers to an edge barrier (or wicking layer) that comprises a material that will substantially prevent the passage of absorbent particulate polymer material through it.

In the preferred embodiment, the edge barrier is substantially liquid impermeable to prevent sideways leakage of fluid from the absorbent structure. By providing an absorbent article with an absorbent structure that has a substantially liquid impermeable edge barrier prevents that liquid in the absorbent structure, which is unable to be absorbed in time by the absorbent material in the absorbent structure, can go beyond the side edge(s), front edge and/or back edge of the absorbent structure and thus can wet, soil and contact the undergarment of the wearer of the absorbent article. Moreover, such edge barrier also helps to block, transport and/or redistribute the liquid in the absorbent article, as the liquid can flow along the edge barrier to other parts of the absorbent structure where it is able to be absorbed by absorbent material not yet saturated with liquid.

In the preferred embodiment, the edge barrier is substantially absorbent particulate polymer material impermeable to prevent sideways leakage of absorbent particulate polymer material from the absorbent structure. Such substantially absorbent particulate polymer material impermeable edge barrier prevents absorbent particulate material from escaping along the side edge(s), front edge and/or back edge of the absorbent structure, both in dry and in wet state, thus aiding in improving the immobilization of the absorbent material within the absorbent structure. It also prevents that absorbent particulate polymer material can come in contact with the wearer which can cause irritation, skin problems and overall discomfort for the user. Furthermore, such edge barrier provides an extra protection against ruptures or pinholes created through the liquid pervious topsheet and/or liquid impervious backsheet of the absorbent article during manufacturing, high pressured packaging, transportation and storing, or regular and prolonged usage by the user and/or caregiver.

In the preferred embodiment, the edge barrier is substantially liquid impermeable and substantially absorbent particulate polymer material impermeable to prevent sideways leakage of both fluid and absorbent particulate polymer material from the absorbent structure, allowing both an optimal leakage protection and improved absorbent material immobilization.

The substantially liquid impermeable edge barrier runs along at least a part of at least one of the side edges, along at least a part of the front edge and/or along at least a part of the back edge, which edge barrier extends along at least a part of the thickness of the absorbent structure.

The shape and configuration of the edge barrier at the side edge(s), front edge and/or back edge can be modified depending on various factors such as economic or production reasons or raw material needs. It may, for example, be adapted to comply with the size of the absorbent structure or with the composition and configuration of the absorbent structure, such as the amount of absorbent particulate polymer material and/or fluff pulp within the absorbent structure. It may also be adapted to comply with, for example, flow patterns created in the absorbent structure, or problem or target areas within the absorbent structure. Preferably, the edge barrier extends along the entire thickness of the absorbent structure in order to prevent liquid and/or absorbent particulate polymer material from "spilling" over the edge barrier. Preferably, the edge barrier runs along at least a part of both side edges of the absorbent article, as these side edges are often more prone to sideways liquid and/or absorbent particulate polymer material leakage. In another preferred embodiment, the edge barrier runs along at least a part of both side edges, the front edge and/or the back edge. In a most preferred embodiment, the edge barrier runs along substantially the entire length of the two side edges of the absorbent structure and/or the entire length of the front and/or back edge.

In an embodiment according of the current invention, the edge barrier extends towards the upper layer and/or lower layer of the absorbent structure where it extends along at least a part of the length and width of the absorbent structure in order to provide additional protection against "spillover" or leakage of fluid and/or absorbent particulate polymer material. In one embodiment, the edge barrier extends along only a part of the length and width of the absorbent structure. In another embodiment, the edge barrier extends along the entire length and width of the absorbent structure. The term "upper layer" as used herein refers to the layer or component of the absorbent structure which is positioned nearest to the topsheet; conversely, the term "lower layer" refers to the layer or component of the absorbent structure which is positioned nearest to the backsheet. The edge barrier may, for instance, be at least partially positioned between the topsheet respectively backsheet and the upper layer respectively lower layer of the absorbent structure, or alternatively, between the upper layer respectively lower layer of the absorbent structure and an additional layer(s), positioned between the topsheet respectively backsheet and the absorbent structure. It should be noted that the phrase "extends along at least a part of the length and width of the absorbent structure" used here means that the edge barrier can extend along at least a part of the length and width of the absorbent structure on either side of the upper and/or lower layer of the absorbent structure. If, for example, the lower layer is a wicking layer, the edge barrier can extend either above or below the wicking layer, when looking along the z-axis.

In another embodiment of the current invention, the edge barrier is folded double along the x-axis and/or y-axis, resulting in a double protection barrier against leakage of fluid and/or absorbent particulate polymer material along the edge(s) of the absorbent structure.

Preferably, the edge barrier comprises a substantially continuous layer of foil, film, closed foam, plastic, nonwoven or similar substantially inherently or rendered liquid impermeable and/or absorbent particulate polymer material impermeable materials, media and/or layers. The edge barrier may be provided with a hydrophobic and/or hydrophilic coating. Preferably, the edge barrier is hydrophobic or substantially hydrophobic. With the term "hydrophobic" used here, it is meant that water in contact with the edge barrier will make a water contact angle that is larger than 90°. This hydrophobicity will further contribute to the spreading of fluid along the edge barrier to other parts of the absorbent structure. In a preferred embodiment according to the present invention, the edge barrier is a polyethylene or polypropylene film, more preferably a coated polyethylene or polypropylene film, preferably whereby said film comprises a thickness in the range from 5 to 1500 µm, more preferably from 10 to 500 µm, most preferably from 25 to 150 µm.

In a preferred embodiment of the invention, the absorbent structure further comprises a substantially liquid impermeable and/or substantially absorbent particulate polymer material impermeable wicking layer which faces the backsheet of the absorbent article and extends along at least a part of the length and width of the absorbent structure. With the term "faces the backsheet" it is meant that the wicking layer corresponds with the layer or component of the absorbent structure which is positioned nearest to the backsheet, which layer can be in direct contact with the backsheet or in direct contact with an additional layer(s) positioned in between the absorbent structure and the backsheet. Preferably, the wicking layer extends along the entire width and length of the absorbent structure.

In one embodiment, the wicking layer is substantially liquid impermeable. Such wicking layer prevents liquid from penetrating through the absorbent structure along the z-direction and helps in spreading the liquid across the absorbent structure in the x-y-direction, thereby allowing to more optimally utilize the absorbent capacity of the absorbent structure. Preferably, the wicking layer comprises a hydrostatic head of at least 100 mm, more preferably a hydrostatic head of at least 200 mm, most preferably a hydrostatic head of at least 300 mm.

In another embodiment, the wicking layer is substantially absorbent particulate polymer material impermeable. The wicking layer hereby provides a protective barrier for the absorbent structure to prevent that absorbent material can escape from the absorbent structure along the z-direction.

In a preferred embodiment, the wicking layer is substantially liquid impermeable and substantially absorbent particulate polymer material impermeable so as to prevent both liquid and absorbent particulate polymer material from escaping the absorbent structure along the z-direction.

In a preferred embodiment, the wicking layer comprises a substantially continuous layer of foil, film, closed foam, plastic, nonwoven or similar substantially inherent or rendered liquid-impermeable and/or substantially absorbent particulate polymer material impermeable materials, media and/or layers. The wicking layer may be provided with a hydrophobic and/or hydrophilic coating. Preferably, the wicking layer is hydrophobic or substantially hydrophobic. With the term "hydrophobic" used here, it is meant that water in contact with the wicking layer will make a water contact angle that is larger than 90°. This hydrophobicity will further contribute to the spreading of fluid along the wicking layer to other parts of the absorbent structure. In a preferred embodiment according to the present invention, the wicking layer is a polyethylene or polypropylene film, more preferably a coated polyethylene or polypropylene film, preferably whereby said film comprises a thickness in the range from 5 to 1500 µm, more preferably from 10 to 500 µm, most preferably from 25 to 150 µm.

In a particularly preferred embodiment, the absorbent structure comprises an edge barrier and a wicking layer, which edge barrier and wicking layer are made out of one single piece of material. This means that the edge barrier and the wicking layer are formed from one piece of material which extends from at least one of the edges of the absorbent structure along at least a part of its thickness, hereby providing the absorbent structure with an edge barrier, which material further extends towards the side of the absorbent structure facing the backsheet, where the material extends along at least a part of the length and width of the absorbent structure, hereby providing the wicking layer. The single piece of material can comprise a substantially continuous layer of foil, film, closed foam, plastic and/or nonwoven material, or similar substantially inherently or rendered liquid-impermeable and/or substantially absorbent particulate polymer material impermeable materials, media and/or layers, and/or can comprise similar properties as described above for the edge barrier and wicking layer. In a preferred embodiment, the single piece of material forming the edge barrier and the wicking layer is a polyethylene or polypropylene film, more preferably a coated polyethylene or polypropylene film, preferably whereby said film comprises a thickness in the range from 5 to 1500 µm, more preferably from 10 to 500 µm, most preferably from 25 to 150 µm.

The absorbent structure according to the current invention may further comprise any type of components or layers positioned in any type of configuration known in the art. The absorbent structure may for example comprise absorbent core layers, acquisition and/or distribution layers, surge layers, highloft and/or coverstock layers and the like.

The absorbent structure according to the current invention may comprise any absorbent material that is generally compressible, conformable, non-irritating to the wearer's skin, and capable of absorbing and retaining bodily exudates. The absorbent structure according to the current invention may comprise a wide variety of liquid absorbent materials commonly used in absorbent articles such as fluff pulp, which is generally referred to as airlaid; creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. However, preferably, the use of cellulose fibers or fluff pulp is limited. In a preferred embodiment, the absorbent article is substantially cellulose free. Preferably, the absorbent structure comprises absorbent particulate polymer material as absorbent material.

In a preferred embodiment according to the current invention, the absorbent structure comprises an absorbent core, which absorbent core comprises at least one essentially fluffless fibrous substrate layer in which absorbent particulate polymer material is dispersed and/or embedded. Preferably, the weight ratio of the absorbent particulate polymer material versus the substrate layer is at least 20 %, more preferably at least 30 %. Preferably, the absorbent core has a liquid acquisition, distribution and storage function, whereby the substrate layer not only helps in immobilizing the absorbent particulate polymer material, but also aids in distributing liquid across the absorbent structure, allowing it to be subsequently absorbed by the absorbent particulate polymer material. This further results in a more optimal use of the absorbent capacity of the absorbent structure.

With the term "essentially fluffless fibrous substrate layer" as used herein, it is referred to that the substrate layer contains less than 20% by weight cellulosic fibres or fluff pulp, less than 10% cellulosic fibres or fluff pulp, less than 5% cellulosic fibres or fluff pulp, no cellulosic fibres or fluff pulp, or no more than an immaterial amount of cellulosic fibres or fluff pulp which do not materially affect the thinness, flexibility or absorbency thereof. In a preferred embodiment, the fibrous substrate layer is a thermobonded, airthrough bonded or chemically bonded nonwoven material.

In a preferred embodiment, the absorbent core of the absorbent structure further comprises at least one nonwoven layer. Such nonwoven layer provides an additional barrier for the absorbent core so as to prevent that absorbent particulate polymer material can escape from the absorbent core. In a more preferred embodiment, the absorbent core is positioned in between two nonwoven layers, thus providing a protection barrier on both sides of the absorbent core. One of the two nonwoven layers may also have the same or similar properties as the wicking layer and thus may be uniform therewith, provided that that nonwoven layer is substantially liquid impermeable and/or substantially absorbent particulate polymer material, faces the backsheet of the absorbent article and extends along at least a part of the length and width of the absorbent structure. Besides protecting absorbent material from escaping the absorbent core, at least one of the two nonwoven layers may also have other functions, such as for example the function of an acquisition and/or distribution layer, surge layer, etc. The two nonwoven layers may be at least partially joined together by attachments made up of substantially permanent primary attachments and/or substantially temporary secondary attachments, so as to form a sandwich-like composite structure containing the absorbent core. The substantially temporary secondary attachments may hereby consist of various sub-regions, corresponding with different separation forces. Such substantially permanent primary attachments and/or substantially temporary secondary attachments are for example described in WO 2012/052 172.

The absorbent article according to the present invention may also employ additional layers known in the art positioned in between the topsheet and the absorbent structure and/or positioned in between the backsheet and the absorbent structure, such as acquisition and/or distribution layers, surge layers, highloft and/or coverstock layers and the like.

In another preferred embodiment the current invention provides an essentially fluffless absorbent structure for use in an absorbent article as described above, said absorbent structure comprising a length along an x-axis, a width along a y-axis and a thickness along a z-axis. The absorbent structure further comprises two laterally opposed side edges extending substantially along the x-axis and a front edge and a back edge which extend substantially along the y-axis. In particular, the absorbent structure comprises an edge barrier, which edge barrier is substantially liquid impermeable and/or substantially absorbent particulate polymer material impermeable to prevent sideways leakage of fluid and/or absorbent particulate polymer material from said absorbent structure. The edge barrier is hereby disposed along at least a part of at least one of the side edges, along at least a part of the front edge and/or along at least a part of the back edge, which edge barrier extends along at least a part of the thickness of the absorbent structure. Such edge barrier can have various configurations and may comprise various materials as described above.

In preferred embodiment, the absorbent structure further comprises a substantially liquid impermeable and/or substantially absorbent particulate polymer material impermeable wicking layer extending along at least a part of the length and width of the absorbent structure. The wicking layer can have various configurations and may comprise various materials as described above.

In a particularly preferred embodiment, the absorbent structure comprises an edge barrier and a wicking layer, whereby the edge barrier and wicking layer are made out of one single material. Such single piece of material can have various configurations and may comprise various materials as described above.

The various components or layers of the absorbent structure may be assembled to each other using a wide variety of techniques well known in the art such as, but not limited to, the use of a thermoplastic, adhesive, glue and/or binder, and/or the use of thermal, mechanical, thermo-mechanical and/or ultrasonic processes.

The present invention will be now described in more details, referring to examples that are not intended to, nor should they be interpreted to, limit the scope of the invention.

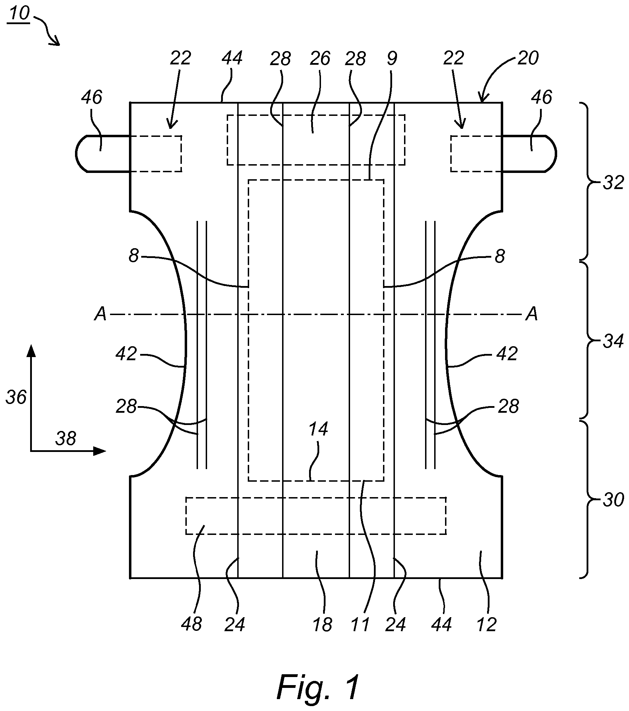

Figure 1 is a top plan view of a diaper 10 as a preferred embodiment of an absorbent article including an absorbent structure 14 according to the present invention. It should be understood, however, that the present invention is also applicable to other absorbent articles such as feminine hygiene garments, baby pants, adult incontinent garments and the like.

The absorbent article is shown in its flat out, un-contracted state with the wearer side facing the viewer. The chassis 12 of the diaper 10 in Figure 1 comprises the main body of the diaper 10. The chassis 12 comprises an outer covering including a liquid pervious topsheet 18 and/or a liquid impervious backsheet 20. The chassis 12 may include a portion of an essentially fluffless absorbent structure 14 encased between the topsheet 18 and the backsheet 20. The chassis 12 may also include most or all of the absorbent structure 14 encased between the topsheet 18 and the backsheet 20. The chassis 12 preferably further includes side panels or ears 22, elasticized leg cuffs 28 and elastic waist features 26. One end portion of the diaper 10 is configured as a front waist region 30 of the diaper 10. The opposite end portion is configured as a back waist region 32 of the diaper 10. An intermediate portion of the diaper 10 is configured as a crotch region 34, which extends longitudinally between the first and second waist regions 30 and 32. The waist regions 30 and 32 may include elastic elements such that they gather about the waist of the wearer to provide improved fit and containment (e.g. elastic waist feature 26). The crotch region 34 is that portion of the diaper 10 which, when the diaper 10 is worn, is generally positioned between the wearer's legs. The diaper 10 is depicted with its longitudinal axis 36 and its transverse axis 38. The periphery of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper. The chassis 12 also comprises a fastening system, which may include at least one fastening or securing member 46 and at least one landing zone 48. The various components within the diaper 10 may be bound, joined or secured by any method known in the art, for example by adhesives in uniform continuous layers, patterned layers or arrays of separate lines, spirals or spots. The topsheet 18, the backsheet 20, the absorbent structure 14 and other components may be assembled in a variety of well-known configurations and are well known in the art.

The backsheet 20 covers the absorbent structure 14 and preferably extends beyond the absorbent structure 14 toward the longitudinal edges 42 and end edges 44 of the diaper 10 and may be joined with the topsheet 18. The backsheet 20 prevents the bodily exudates absorbed by the absorbent structure 14 and contained within the diaper 10 from soiling other external articles that may contact the wearer, such as bed sheets and undergarments. In preferred embodiments, the backsheet 20 is substantially impervious to bodily exudates and comprises a laminate of a nonwoven and a thin plastic film such as a thermoplastic film. The backsheet 20 may comprise breathable materials that permit vapour to escape from the diaper 10 while still preventing bodily exudates from passing through the backsheet 20. It may be semi-rigid, non-elastic and can be made fully or partially elasticized and include backing. The backsheets 20 may be assembled in a variety of well-known configurations and are well known in the art.

The diaper 10 comprises a topsheet 18 that is preferably soft, compliant, exhibits good strikethroughs and has a reduced tendency to rewet from the liquid absorbent material. The topsheet 18 is placed in close proximity to the skin of the wearer when the diaper 10 is worn. In this way, such topsheet 18 permits bodily exudates to rapidly penetrate it so as to flow toward the absorbent structure 14 more quickly, but preferably not allowing such bodily exudates to flow back through the topsheet 18. The topsheet 18 may be constructed from any one of a wide range of liquid and vapour permeable, preferably hydrophilic, materials. The upper and lower surface of the topsheet 18 may be treated differently and may for instance include a surfactant on the upper surface so as to facilitate liquid transfer there through, especially at a central zone or area of the topsheet 18 located over the absorbent structure 14, and for instance include a hydrophobic agent on the lower surface to minimize the liquid contained within the absorbent core from contact wetting the topsheet 18 thereby reducing rewet values. The topsheet 18 may also be coated with a substance having rash preventing or rash reducing properties (e.g. aloe vera). The topsheet 18 covers substantially the entire wearer facing area of the diaper 10, including substantially all of the front waist region 30, back waist region 32, and crotch region 34. Further, the side panels 22 and/or waist feature layers of the inner region may be formed from the same single topsheet material and, thus, may be referred to as being unitary with the topsheet 18 in forming longitudinal and lateral extensions of the topsheet 18 material. Alternatively, the topsheet 18 may be formed from multiple different materials which vary across the width of the topsheet 18. Such a multiple piece design allows for creation of preferred properties and different zones of the topsheet 18. The topsheet 18 be semi-rigid, non-elastic and can be made fully or partially elasticized. The topsheet 18 may be assembled in a variety of well-known configurations and are well known in the art.

The essentially fluffless absorbent structure 14 in Figure 1 generally is disposed between the topsheet 18 and the backsheet 20. The absorbent structure 14 may comprise any absorbent material that is generally compressible, conformable, non-irritating to the wearer's skin, and capable of absorbing and retaining bodily exudates. The absorbent structure 14 may comprise a wide variety of liquid absorbent materials commonly used in absorbent articles such as fluff pulp, which is generally referred to as airlaid. Examples of other suitable absorbent materials include creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. However, preferably, the use of cellulose fibers and fluff pulp is limited. The absorbent structure 14 may further comprise minor amounts (typically less than 10 %) of non-liquid absorbent materials, such as adhesives, binders, plastics, waxes, oils and the like. The absorbent structure 14 according to various embodiments of the invention may be configured to extend substantially the full length and/or width of the diaper 10. However, alternatively the absorbent structure 14 according to the invention is not coextensive with the entire diaper 10 and is limited to certain regions of the diaper 10 such as for instance the crotch region 34. In various embodiments, the absorbent structure 14 extends to the edges of the diaper 10 and the absorbent material is concentrated in the crotch region 34 or another target zone of the diaper 10. In still another embodiment, the particles can be a combination of absorbent material, preferably comprising absorbent polymer material, and skin care particles such as ion exchange resins, deodorant, anti-microbial agents, binder particles, or other beneficial particles. The absorbent structure 14 is displayed as having a substantially rectangular configuration, however, the absorbent structure can also comprise other shapes known in the art such as, elliptical shaped, dogbone shaped, T-shaped or I-shaped configurations.

The diaper 10 may also utilize a pair of containment walls or cuffs 24. Each cuff 24 is a longitudinally extending wall structure preferably positioned on each side of the absorbent structure 14 and spaced laterally from the longitudinal axis 36. The longitudinal ends of the cuffs 24 may be attached or joined, for example, to the topsheet 18 in the front and rear waist regions 30 and 32. Preferably, the ends of the cuffs 24 are tacked down inwardly and attached, for example, by adhesive or sonic bonding to the lower structure. Such a construction effectively biases the cuffs 24 inwardly and is generally considered to cause the cuffs 24 to exhibit improved leakage prevention properties. Preferably, the cuffs 24 are equipped with elastic members 28, which extend along a substantial length of the cuffs 24. In a common application, the elastic members 28 are placed within the cuffs 24, preferably at the top of the cuff 24 while in a stretched condition and then glued or sonic bonded to the cuff 24 at least at their ends. When released or otherwise allowed relaxing, the elastic members 28 retract inwardly. When the diaper 10 is worn, the elastic members 28 function to contract the cuffs 24 about the buttocks and the thighs of the wearer in a manner, which forms a seals between the diaper 10, the buttocks and the thighs. The cuffs 24 may be assembled in a variety of well-known configurations and are well known in the art.

The diaper 10 may also employ additional layers known in the art including an acquisition layer or surge layer, preferably situated between the topsheet and the absorbent core and highloft and/or coverstock layers. This serves to slow down the flow so that the liquid has adequate time to be absorbed by the absorbent core.

In order to keep the diaper 10 in place about the wearer, preferably at least a portion of the back waist region 32 is attached by fastening or securing members 46 to at least a portion of the front waist region 30, preferably to form leg openings and an absorbent article waist. Fastening or securing members 46 carry the tensile load around the absorbent article waist and compliment the elastic members 28 by providing a quasi-seal between the wearer, the elastic waist feature 26 and cuffs 24, so that bodily exudates are contained within the diaper 10 which are then absorbed. In other words, so that it does not leak through gaps between the wearer and the edge of the diaper 10. The fastening or securing members 46 may for instance be adhesive, mechanical fasteners, hook and loop features, conceivable strings and/or combinations thereof, i.e., anything that will secure one end of the diaper 10 to the longitudinally opposite end of the diaper 10. The fastening or securing members 46 may also be co-adhesive such that they adhere to each other but not other materials. The fastening or securing members 46 and any component thereof may include any material suitable for such a use, including but not limited to plastics, films, foams, non-woven webs, woven webs, paper, laminates, fibre reinforced plastics and the like, or combinations thereof. It may be preferable that the materials making up the fastening or securing members 46 are flexible, extensible and/or elastic, allowing them to better conform to the shape and movements of the body and thus, reduces the likelihood that the fastening system will irritate or injure the wearer's skin. Preferably, the diaper 10 is affixed to the wearer by tape fasteners which are permanently affixed to the backsheet 20. Tape fasteners are contacted with the transversely opposite side panel or ears 22 attached or joined and extending from the backsheet 20, where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening and securing elements 46 may be assembled in a variety of well-known configurations and are well known in the art.

The waist regions 30 and 32 each comprise a central region and a pair of side panels or ears 22 which typically comprise the outer lateral portions of the waist regions. These side panels 22 may be unitary with the chassis 12 and/or backsheet 20 or may be attached or joined thereto by any means know in the art. In a preferred embodiment of the present invention, the side panels 22 positioned in the back waist region 32 are flexible, extensible and/or elastic in at least the lateral direction (i.e., elasticized side panels), in another embodiment the side panels 22 are non-elastic, semi-rigid, rigid and/or stiff. These variety of side panels 22 are well known in the art.

Furthermore waistbands 26 employing elastic members can be positioned along the transverse portion of the diaper 10 so that when worn, the waistbands 26 are positioned along the waist of the wearer. Generally, the waistband 26 preferably creates a seal against the waist so that bodily exudates do not leak from the regions between the elastic waistband 26 and the waist of the wearer. Although the bodily exudates are primarily absorbed by the absorbent materials within the diaper 10, the seal is important considering the assault of liquid by the wearer may overwhelm the absorption rate capacity of the absorbent structure 14. Hence, the waistbands 26 contain the liquid while it is being absorbed, they are well known in the art.

The absorbent article such as a diaper 10 may also include such other features, components and elements as are known in the art including front and rear ear panels, waist cap features, elastics and the like to provide better fit, containment and aesthetic characteristics. These features may be assembled in a variety of well-known configurations and are well known in the art.

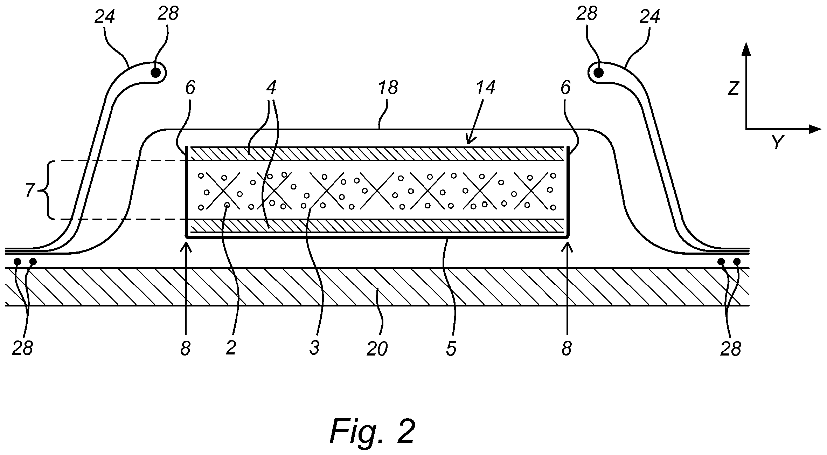

Figure 2 shows a schematic view of the absorbent article of Figure 1 cut along line A-A, hereby showing the various components of the essentially fluffless absorbent structure 14 according to a preferred embodiment of the current invention.

As is shown in Figure 2, the absorbent structure 14 is positioned in between the topsheet 18 and the backsheet 20. The absorbent structure according to this embodiment comprises an absorbent core 7 positioned in between two nonwoven layers 4. The absorbent core hereby comprises absorbent particulate polymer material, schematically represented by the dots 2 in Figure 2, which absorbent particulate polymer material is dispersed in a fluffless fibrous substrate layer, schematically represented by the X's 3 in Figure 2. The two nonwoven layers 4 provide a first barrier to prevent that the absorbent particulate polymer material can escape from the absorbent core. According to the embodiment displayed in Figure 2, the absorbent structure comprises two liquid impermeable edge barriers 6 along the side edges 8 of the absorbent structure 14 so as to prevent liquid and/or absorbent particulate polymer material from leaking along these edges. As further can be seen in Figure 2, the edge barrier is made from a single piece of a material which also forms a wicking layer 5, which is positioned facing towards the backsheet and prevents leakage of liquid and/or absorbent particulate material along the z-axis and provides a layer to distribute liquid and/or absorbent particulate polymer material along the x-y plane of the absorbent structure, hereby optimally utilizing the absorbent capacity of the absorbent structure. Further, leg elastics 28 are shown which are positioned between the topsheet 18 and the backsheet 20, as well as the containment cuffs 24 which are held in an upward position by elastic members 28.

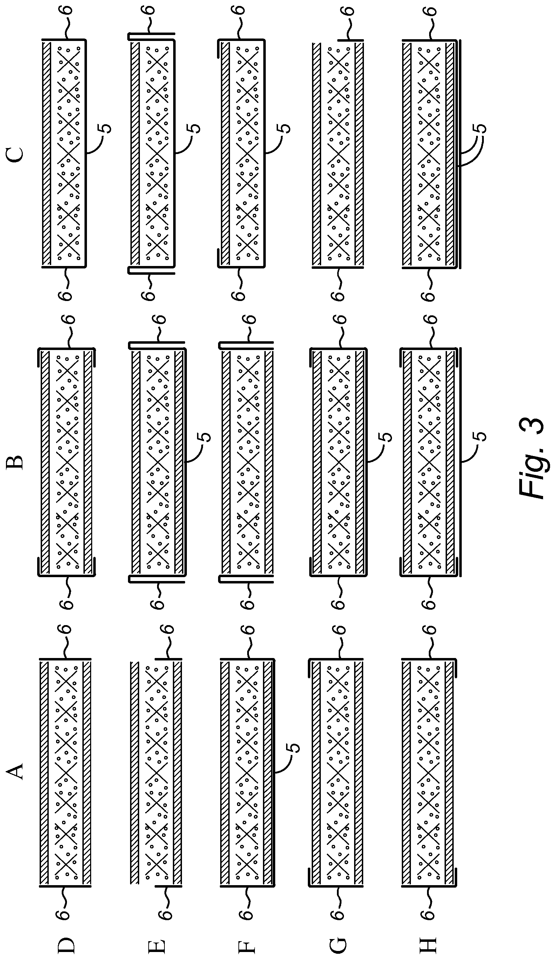

Figure 3 displays cross-sectional schematic illustrations of absorbent structures in the x-z plane and/or y-z plane according to various embodiments of the current invention, with reference number 6 indicating the edge barrier and reference number 5 indicating the wicking layer. As shown in figure 3, the edge barrier can extend along the entire thickness of the absorbent structure (for example AD) or only part thereof (for example AE or CG). The edge barrier can also extend towards the upper layer and/or lower layer of the absorbent structure, where it extends along at least a part of the length and width of the absorbent structure, such as for example depicted in AG, AH or BD. The edge barrier can also be folded double along the x-axis and/or y-axis, such as for example depicted in BE, BF and CE, hereby providing the absorbent structure with a double protection barrier. Further, the absorbent structure can contain a wicking layer which can be separate from the edge barrier (for example BH, CH) or can be made out made out of one single piece of material together with the edge barrier (for example BE, AF, BG, CD, CE or CF). The wicking layer may also be unitary with one of the two nonwoven layers in de absorbent structure, as for example depicted in CD, CE and CF. It should be noted that the configuration of the absorbent structure, edge barrier and/or wicking layer is not limited to these examples.

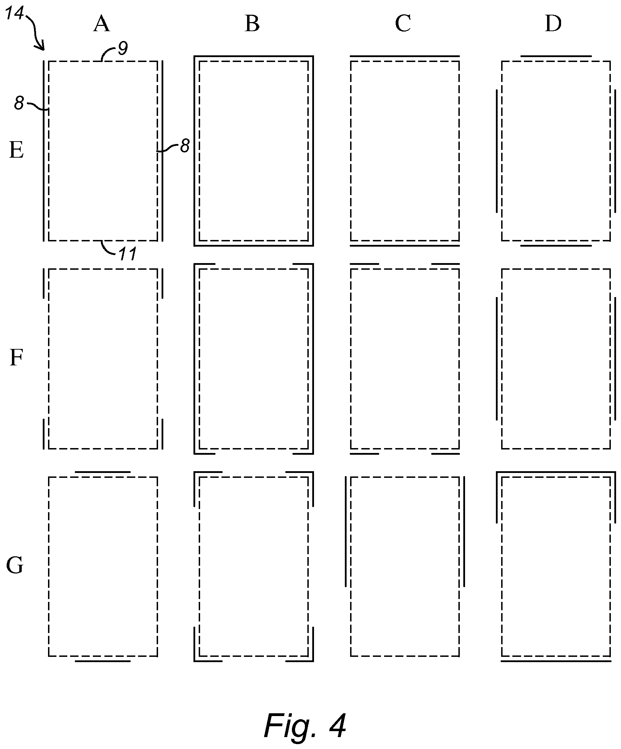

Figure 4 displays a schematic top plan view of a few of the possible shapes and configurations for the edge barrier (depicted by the solid line) in an absorbent structure (depicted by the dotted line) in the x-y plane. Though the absorbent structure in Figure 4 is depicted as having a general rectangular configuration, the absorbent structure can also comprise other shapes known in the art such as, elliptical shaped, dogbone shaped, T-shaped or I-shaped configurations, etc. The absorbent structure 14 comprises two side edges 8 and a front edge 11 and a back edge 9. The edge barrier can be disposed along the entire length of the side edges 8 of the absorbent structure 14 (AE, BE or BF) or only part thereof (AF, BG, CG, DE, DF or DG). Similarly, the edge barrier can be disposed along the entire length of the front and/or back edge of the absorbent structure 14 (BE, CE or DG) or only part thereof (AG, BF, BG, CF or DE). The edge barrier can be disposed along at least a part of only the side edges 8 (AE, AF or CG), along at least a part of only the front 11 and/or back edge 9 (AG, CE or CF) or disposed along at least a part of the side edges 8, front edge 11 and/or back edge 9 (BE, BF, BG, DE or DG). It should be noted that the configuration of the edge barrier is not limited to these examples.

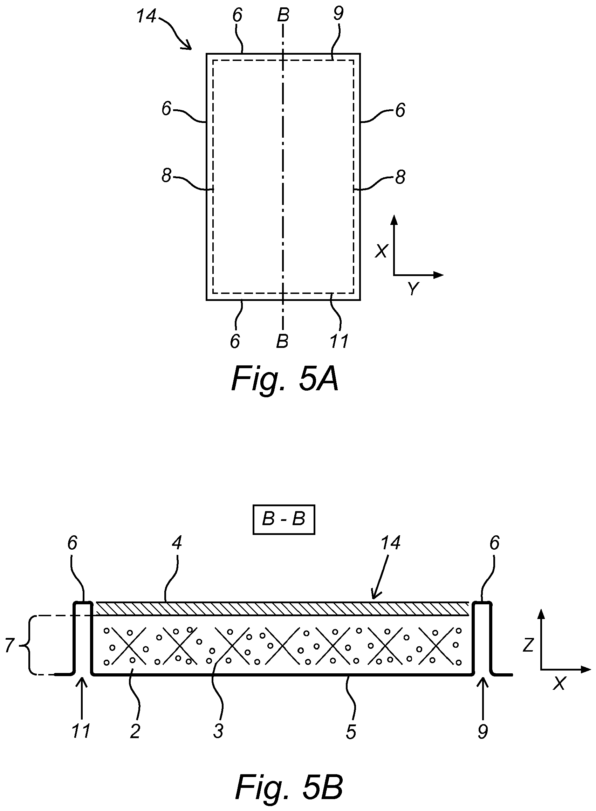

Figure 5A shows a preferred embodiment according to the present invention, whereby the edge barrier 6 of the absorbent structure is provided along the entire length of the side edges 8 and the front edge 11 and back edge 9 of the essentially fluffless absorbent structure 14. The schematic view of a cut made along line B-B in such absorbent structure is displayed in Figure 5B. The absorbent structure hereby comprises an absorbent core 7 positioned in between a nonwoven layer 4 and a wicking layer 5, with the absorbent core 7 comprising absorbent particulate polymer material 2 dispersed in a fluffless fibrous substrate layer 3. Also displayed are the edge barriers 6 along the front edge 11 and back edge 9 of the absorbent structure 14 so as to prevent liquid and/or absorbent particulate material from leaking along these edges. The edge barrier is hereby made from a single piece of material which also forms the wicking layer 5, which prevents leakage of liquid and/or absorbent particulate material along the z-axis and provides a layer to distribute liquid and/or absorbent particulate material along the x-y plane of the absorbent structure. In this embodiment, the edge barrier is folded double along the y-axis in the form of a loop, providing a double protection barrier.

## Claims

1. An absorbent article comprising a liquid pervious topsheet (18), a liquid impervious backsheet (20), and an essentially fluffless absorbent structure (14) positioned in between said backsheet and said topsheet, said absorbent structure comprising a length along an x-axis, a width along a y-axis and a thickness along a z-axis, whereby said absorbent structure comprises two laterally opposed side edges (8) extending substantially along the x-axis and a front edge (11) and a back edge (9) which extend substantially along the y-axis, wherein the absorbent structure further comprises an edge barrier (6), said edge barrier being disposed along at least a part of at least one of the side edges, along at least a part of the front edge and/or along at least a part of the back edge, wherein the edge barrier (6) is substantially absorbent particulate polymer material (2) impermeable to prevent sideways leakage of absorbent particulate polymer material from said absorbent structure **characterized in that** the edge barrier (6) extends along the entire thickness of the absorbent structure and the edge barrier (6) is substantially liquid impermeable to prevent sideways leakage of fluid from said absorbent structure.

2. Absorbent article according to claim 1, whereby the edge barrier (6) extends towards an upper layer and/or lower layer of the absorbent structure, where it extends along at least a part of the length and width of the absorbent structure.

3. Absorbent article according to claim 1 or 2, whereby the edge barrier (6) is folded double along the x-axis and/or y-axis.

4. Absorbent article according to any of claims 1-3, whereby the edge barrier (6) is disposed along at least a part of both side edges.

5. Absorbent article according to any of claims 1-4, whereby the edge barrier (6) is disposed along at least a part of both side edges, the front edge and/or the back edge.

6. Absorbent article according to any of the previous claims, whereby the edge barrier (6) comprises a hydrostatic head of at least 100 mm as defined in the description.

7. An absorbent article according to any of the previous claims, whereby the absorbent structure comprises a substantially liquid impermeable and/or substantially absorbent particulate polymer material impermeable wicking layer (5), which wicking layer faces the backsheet of the absorbent article and extends along at least a part of the length and width of the absorbent structure.

8. An absorbent article according to claim 7, **characterized in that** the wicking layer (5) comprises a hydrostatic head of at least 100 mm.

9. An absorbent article according to claims 7 or 8, whereby the wicking layer (5) and the edge barrier (6) are made out of one single piece of material.

10. An absorbent article according to claim 9, whereby said single piece of material comprises a substantially continuous layer of foil, film, closed foam, plastic and/or nonwoven material.

11. Absorbent article according to any of the previous claims, whereby the absorbent structure comprises an absorbent core (7), which absorbent core comprises at least one essentially fluffless fibrous substrate layer (3) in which absorbent particulate polymer material is dispersed and/or embedded.

12. Absorbent article according to claim 11, whereby the weight ratio of the absorbent particulate polymer material (2) versus the substrate layer is at least 20 %.

13. Absorbent article according to claim 11 or 12, whereby said essentially fluffless fibrous substrate layer is a thermobonded, airthrough bonded or chemically bonded nonwoven material.

14. An absorbent article according to any of the previous claims 11-13, whereby the absorbent structure further comprises at least one nonwoven layer (4).

15. An absorbent article according to claim 14, whereby the absorbent core (7) is positioned in between two nonwoven layers.

16. An essentially fluffless absorbent structure (14) for use in an absorbent article according to any of the previous claims, said absorbent structure comprising a length along an x-axis, a width along a y-axis and a thickness along a z-axis, whereby said absorbent structure comprises two laterally opposed side edges (8) extending substantially along the x-axis and a front edge (11) and a back edge (9) which extend substantially along the y-axis, wherein the absorbent structure further comprises an edge barrier (6), said edge barrier being disposed along at least a part of at least one of the side edges and/or along at least a part of the front edge and/or along at least a part of the back edge, wherein the edge barrier (6) is substantially absorbent particulate polymer material (2) impermeable to prevent sideways leakage of absorbent particulate polymer material from said absorbent structure **characterized in that** the edge barrier (6) extends along the entire thickness of the absorbent structure and the edge barrier (6) is substantially liquid impermeable to prevent sideways leakage of fluid from said absorbent structure.

17. Absorbent structure according to claim 16, whereby the absorbent structure comprises a substantially liquid impermeable and/or substantially absorbent particulate polymer material impermeable wicking layer (5) extending along at least a part of the length and width of the absorbent structure.

18. Absorbent structure according to claim 17, whereby the wicking layer (5) and the edge barrier (6) are made out of one single piece of material.

## Patentansprüche

1. Absorbierender Artikel, der eine flüssigkeitsdurchlässige oberste Lage (18), eine flüssigkeitsundurchlässige Unterlage (20) und eine im Wesentlichen fusselfreie absorbierende Struktur (14), die zwischen der Unterlage und der obersten Lage positioniert ist, aufweist, wobei die absorbierende Struktur eine Länge entlang einer x-Achse, eine Breite entlang einer y-Achse und eine Dicke entlang einer z-Achse aufweist, wobei die absorbierende Struktur zwei seitlich entgegengesetzte Seitenkanten (8), die sich im Wesentlichen entlang der x-Achse erstrecken, und eine Vorderkante (11) und eine Hinterkante (9), die sich im Wesentlichen entlang der y-Achse erstrecken, aufweist, wobei die absorbierende Struktur ferner eine Kantenbarriere (6) aufweist, wobei die Kantenbarriere entlang wenigstens eines Teils wenigstens einer der Seitenkanten, entlang wenigstens eines Teils der Vorderkante und/oder entlang wenigstens eines Teils der Hinterkante angeordnet ist, wobei die Kantenbarriere (6) im Wesentlichen absorbierendes partikelförmiges Polymermaterial (2) ist, das undurchlässig ist, um das seitliche Auslaufen von absorbierendem partikelförmigem Polymermaterial aus der absorbierenden Struktur zu verhindern, **dadurch gekennzeichnet, dass** die Kantenbarriere (6) sich entlang der gesamten Dicke der absorbierenden Struktur erstreckt und die Kantenbarriere (6) im Wesentlichen flüssigkeitsundurchlässig ist, um das seitliche Auslaufen von Fluid aus der absorbierenden Struktur zu verhindern.

2. Absorbierender Artikel nach Anspruch 1, wobei die Kantenbarriere (6) sich in Richtung einer oberen Schicht und/oder unteren Schicht der absorbierenden Struktur erstreckt, wobei sie sich entlang wenigstens eines Teils der Länge und Breite der absorbierenden Struktur erstreckt.

3. Absorbierender Artikel nach Anspruch 1 oder 2, wobei die Kantenbarriere (6) entlang der x-Achse und/oder y-Achse zweifach gefaltet ist.

4. Absorbierender Artikel nach einem der Ansprüche 1 - 3, wobei die Kantenbarriere (6) entlang wenigstens eines Teils beider Seitenkanten angeordnet ist.

5. Absorbierender Artikel nach einem der Ansprüche 1 - 4, wobei die Kantenbarriere (6) entlang wenigstens eines Teils beider Seitenkanten, der Vorderkante und/oder der Hinterkante angeordnet ist.

6. Absorbierender Artikel nach einem der vorhergehenden Ansprüche, wobei die Kantenbarriere (6) ein hydrostatisches Gefälle von wenigstens 100 mm, wie in der Beschreibung definiert, aufweist.

7. Absorbierender Artikel nach einem der vorhergehenden Ansprüche, wobei die absorbierende Struktur eine im Wesentlichen flüssigkeitsundurchlässige und/oder im Wesentlichen undurchlässige Saugschicht (5) aus partikelförmigem Polymermaterial aufweist, wobei die Saugschicht der Unterlage des absorbierenden Artikels zugewandt ist und sich entlang wenigstens eines Teils der Länge und Breite der absorbierenden Struktur erstreckt.

8. Absorbierender Artikel nach Anspruch 7, **dadurch gekennzeichnet, dass** die Saugschicht (5) ein hydrostatisches Gefälle von wenigstens 100 mm aufweist.

9. Absorbierender Artikel nach den Ansprüchen 7 oder 8, wobei die Saugschicht (5) und die Kantenbarriere (6) aus einem einzigen Materialstück hergestellt sind.

10. Absorbierender Artikel nach Anspruch 9, wobei das einzige Materialstück eine im Wesentlichen kontinuierliche Schicht aus Folie, einer Dünnschicht, geschlossenem Schaum, Kunststoff und/oder Vliesmaterial aufweist.

11. Absorbierender Artikel nach einem der vorhergehenden Ansprüche, wobei die absorbierende Struktur einen absorbierenden Kern (7) aufweist, wobei der absorbierende Kern wenigstens eine im Wesentlichen fusselfreie faserige Substratschicht (3) aufweist, in der absorbierendes partikelförmiges Polymermaterial verteilt und/oder eingebettet ist.

12. Absorbierender Artikel nach Anspruch 11, wobei das Gewichtsverhältnis des absorbierenden partikelförmigen Polymermaterials (2) gegenüber der Substratschicht wenigstens 20% beträgt.

13. Absorbierender Artikel nach Anspruch 11 oder 12, wobei die im Wesentlichen fusselfreie faserige Substratschicht ein wärmeverbundenes, luftdurchlässig verbundenes oder chemisch verbundenes Vliesmaterial ist.

14. Absorbierender Artikel nach einem der vorhergehenden Ansprüche 11 - 13, wobei die absorbierende Struktur ferner wenigstens eine Vliesschicht (4) aufweist.

15. Absorbierender Artikel Anspruch 14, wobei der absorbierende Kern (7) zwischen zwei Vliesschichten positioniert ist.

16. Im Wesentlichen fusselfreie absorbierende Struktur (14) zur Verwendung in einem absorbierenden Artikel nach einem der vorhergehenden Ansprüche, wobei die absorbierende Struktur eine Länge entlang einer x-Achse, eine Breite entlang einer y-Achse und eine Dicke entlang einer z-Achse aufweist, wobei die absorbierende Struktur zwei seitlich entgegengesetzte Seitenkanten (8), die sich im Wesentlichen entlang der x-Achse erstrecken, und eine Vorderkante (11) und eine Hinterkante (9), die sich im Wesentlichen entlang der y-Achse erstrecken, aufweist, wobei die absorbierende Struktur ferner eine Kantenbarriere (6) aufweist, wobei die Kantenbarriere entlang wenigstens eines Teils wenigstens einer der Seitenkanten und/oder entlang wenigstens eines Teils der Vorderkante und/oder entlang wenigstens eines Teils der Hinterkante angeordnet ist, wobei die Kantenbarriere (6) im Wesentlichen absorbierendes partikelförmiges Polymermaterial (2) ist, das undurchlässig ist, um das seitliche Auslaufen von absorbierendem partikelförmigem Polymermaterial aus der absorbierenden Struktur zu verhindern, **dadurch gekennzeichnet, dass** die Kantenbarriere (6) sich entlang der gesamten Dicke der absorbierenden Struktur erstreckt und die Kantenbarriere (6) im Wesentlichen flüssigkeitsundurchlässig ist, um das seitliche Auslaufen von Fluid aus der absorbierenden Struktur zu verhindern.

17. Absorbierende Struktur nach Anspruch 16, wobei die absorbierende Struktur eine im Wesentlichen flüssigkeitsundurchlässige und/oder im Wesentlichen undurchlässige Saugschicht (5) aus partikelförmigem Polymermaterial aufweist, die sich entlang wenigstens eines Teils der Länge und Breite der absorbierenden Struktur erstreckt.

18. Absorbierende Struktur nach Anspruch 17, wobei die Saugschicht (5) und die Kantenbarriere (6) aus einem einzigen Materialstück hergestellt sind.

## Revendications

1. Article absorbant comprenant une feuille supérieure perméable au liquide (18), une feuille arrière imperméable au liquide (20), et une structure absorbante sensiblement non pelucheuse (14) positionnée entre ladite feuille arrière et ladite feuille supérieure, ladite structure absorbante présentant une longueur suivant un axe x, une largeur suivant un axe y et une épaisseur suivant un axe z, de telle sorte que ladite structure absorbante comprend deux bords latéraux latéralement opposés (8) s'étendant sensiblement suivant l'axe x et un bord avant (11) et un bord arrière (9) qui s'étendent sensiblement suivant l'axe y, dans lequel la structure absorbante comprend, en outre, une barrière de bord (6), ladite barrière de bord étant disposée suivant au moins une partie d'au moins l'un des bords latéraux, suivant au moins une partie du bord avant et/ou suivant au moins une partie du bord arrière, dans lequel la barrière de bord (6) est en un matériau polymère particulaire sensiblement absorbant (2) imperméable afin d'empêcher une fuite latérale de matériau polymère particulaire absorbant à partir de ladite structure absorbante **caractérisé en ce que** la barrière de bord (6) s'étend suivant la totalité de l'épaisseur de la structure absorbante et la barrière de bord (6) est sensiblement imperméable au liquide afin d'empêcher la fuite latérale de fluide à partir de ladite structure absorbante.

2. Article absorbant selon la revendication 1, de telle sorte que la barrière de bord (6) s'étend vers une couche supérieure et/ou une couche inférieure de la structure absorbante, dans laquelle elle s'étend suivant au moins une partie de la longueur et de la largeur de la structure absorbante.

3. Article absorbant selon la revendication 1 ou 2, de telle sorte que la barrière de bord (6) est doublement repliée suivant l'axe x et/ou l'axe y.

4. Article absorbant selon l'une quelconque des revendications 1 à 3, de telle sorte que la barrière de bord (6) est disposée le long d'au moins une partie des deux bords latéraux.

5. Article absorbant selon l'une quelconque des revendications 1 à 4, de telle sorte que la barrière de bord (6) est disposée le long d'au moins une partie des deux bords latéraux, du bord avant et/ou du bord arrière.

6. Article absorbant selon l'une quelconque des revendications précédentes, de telle sorte que la barrière de bord (6) présente une hauteur hydrostatique d'au moins 100 mm tel que défini dans la description.

7. Article absorbant selon l'une quelconque des revendications précédentes, de telle sorte que la structure absorbante comprend une couche d'absorption imperméable de matériau polymère particulaire sensiblement imperméable au liquide et/ou sensiblement absorbante (5), laquelle couche d'absorption fait face à la feuille arrière de l'article absorbant et s'étend le long d'au moins une partie la longueur et de la largeur de la structure absorbante.

8. Article absorbant selon la revendication 7, **caractérisé en ce que** la couche d'absorption (5) présente une hauteur hydrostatique d'au moins 100 mm.

9. Article absorbant selon les revendications 7 ou 8, de telle sorte que la couche d'absorption (5) et la barrière de bord (6) sont fabriquées en un matériau d'une seule pièce.

10. Article absorbant selon la revendication 9, de telle sorte que ledit matériau d'une seule pièce comprend une couche sensiblement continue de matériau en feuille, film, mousse à cellules fermées, matière plastique et/ou matériau non tissé.

11. Article absorbant selon l'une quelconque des revendications précédentes, de telle sorte que la structure absorbante comprend un cœur absorbant (7), lequel cœur absorbant comprend au moins une couche de substrat fibreux sensiblement non pelucheux (3) dans laquelle un matériau polymère particulaire absorbant est dispersé et/ou noyé.

12. Article absorbant selon la revendication 11, de telle sorte que le rapport en poids du matériau polymère particulaire absorbant (2) par rapport à la couche de substrat est d'au moins 20%.

13. Article absorbant selon la revendication 11 ou 12, de telle sorte que la couche de substrat fibreux sensiblement non pelucheux est en un matériau non tissé thermosoudé, soudé à l'air chaud ou soudé chimiquement.

14. Article absorbant selon l'une quelconque des revendications précédentes 11 à 13, de telle sorte que la structure absorbante comprend, en outre, au moins une couche non tissée (4).

15. Article absorbant selon la revendication 14, de telle sorte que le cœur absorbant (7) est positionné entre deux couches non tissées.

16. Structure absorbante sensiblement non pelucheuse (14) destinée à être utilisée dans un article absorbant selon l'une quelconque des revendications précédentes, ladite structure absorbante présentant une longueur suivant un axe x, une largeur suivant un axe y et une épaisseur suivant un axe z, de telle sorte que ladite structure absorbante comprend deux bords latéraux latéralement opposés (8) s'étendant sensiblement suivant l'axe x et un bord avant (11) et un bord arrière (9) qui s'étendent sensiblement suivant l'axe y, dans laquelle la structure absorbante comprend, en outre, une barrière de bord (6), ladite barrière de bord étant disposée suivant au moins une partie d'au moins l'un des bords latéraux, et/ou suivant au moins une partie du bord avant et/ou suivant au moins une partie du bord arrière, dans laquelle la barrière de bord (6) est en matériau polymère particulaire sensiblement absorbant (2) imperméable afin d'empêcher une fuite latérale de matériau polymère particulaire absorbant à partir de ladite structure absorbante **caractérisée en ce que** la barrière de bord (6) s'étend suivant la totalité de l'épaisseur de la structure absorbante et la barrière de bord (6) est sensiblement imperméable au liquide afin d'empêcher la fuite latérale de fluide à partir de ladite structure absorbante.

17. Structure absorbante selon la revendication 16, de telle sorte que la structure absorbante comprend une couche d'absorption imperméable de matériau polymère particulaire sensiblement imperméable au liquide et/ou sensiblement absorbante (5) s'étendant suivant au moins une partie de la longueur et de la largeur de la structure absorbante.

18. Structure absorbante selon la revendication 17, de telle sorte que la couche d'absorption (5) et la barrière de bord (6) sont fabriquées en un matériau d'une seule pièce.
